# EUROPEAN PATENT APPLICATION

(11) **EP 3 058 904 A1**
(43) Date of publication of application: **24.08.2016**
(21) Application number: 16155573.5
(22) Date of filing: 12.02.2016
(51) Int. Cl.: A61F 5/058

(54) **HAND BRACE**

(30) Priority: 17.02.2015 IT FI20150009 U
(71) Applicant: Azienda Ospedaliero-Universitaria Meyer, 50139 Firenze (IT)
(72) Inventor: BASCIANO, Simone, 50065 Pontassieve (Firenze) (IT)
(74) Representative: Brazzini, Silvia

(57) **Abstract**

The present invention refers to the field of orthotic devices and in particular it concerns a brace for a hand that allows effective immobilisation of the fingers in the treatment of trauma, fractures, sprains, strains and the like, which is easy to apply and manage, has high comfort for the patient, particularly suitable for use in patients of paediatric age.

## Description

The present invention refers to the field of orthotic devices and in particular concerns a brace for a hand that allows effective immobilisation of the different fingers of the hand except for the thumb, in the treatment of trauma, fractures, sprains, strains and the like, particularly suitable for use in patients of paediatric age.

Trauma to the upper limbs, especially for those who play sport, is extremely frequent and, if not treated quickly and correctly, can take very long treatment times before being able to completely recover the functionality of the hand, or can cause collateral injuries, and other complications. Even when the trauma does not require surgery, it is also of fundamental importance, in order to accelerate and maximise the restoration of functionality, to take care of immobilising the injured part as soon as possible for protection both to put the healing joint at rest and more generally prevent undesired movements, and to protect possible wounds and stitches.

Currently, various types of orthotic devices are known for immobilising the hand with post-trauma corrective and therapeutic function, which however have one or more drawbacks that actually make their application to the patient problematic and/or can cause secondary damage to the limb, to the soft tissues and/or to micro-circulation, or even do not allow good functional recovery of the mobility of the hand in short time periods.

An example of known orthotic devices is the so-called aluminium Zimmer splint, usually applied with a partial plaster cast to form a so-called "mould cast". This device, whose application necessarily requires the intervention of two operators, can cause compression injuries on the patient and, particularly in children, is difficult to manage since the splint breaks easily generating sharp edges and therefore sources of risk. Therefore the device, although widely used, has two orders of problems related both to application and to the effect on the patient.

Another orthotic device in use that requires the intervention of two operators is the plaster splint; this device also has the risk of breaking. In addition, the plaster splint, in order to anchor it, involves blocking many joints (for example, the fracture of a proximal phalanx of the hand results in the immobilisation of the patient up to the elbow!), with major consequences in terms of convenience for the patient who is moreover forced to wear the device for very long time periods before obtaining satisfactory functional recovery.

The resin splint, another orthotic device currently in use, solves the problem of the risk of breaking of the plaster splint, but it keeps all of the other drawbacks, long functional recovery time, blocking of many joints for anchoring and the need for two operators for application.

Moreover, these types of known orthotic devices prevent adequate home management particularly when there are wounds on the injured part that need frequent medication; in these cases, the patient is indeed forced to go for outpatient care since this type of device can only be removed and applied again by a healthcare worker.

In light of what outlined above, it is clear how orthotic devices known up to now have clear drawbacks and in the field there is a great need to come up with a new device that overcomes such drawbacks and meets the frequent need in orthopaedic and trauma medicine for such a device for immobilising the hand.

The present invention therefore aims to provide a brace with a new configuration, which achieves particular usefulness both in terms of therapeutic effectiveness and validity towards injuries and also in terms of practicality in the first application and in the subsequent management of the brace by the patient.

This result, with the related new advantages, is achieved by the brace according to the invention, whose essential characteristics are defined by the first of the attached claims. Further important characteristics are defined in the dependent claims.

The invention will now be illustrated in greater detail in the following description of an embodiment thereof given as a non-limiting example with reference to the attached drawings in which a brace according to the invention is shown, worn on a hand to highlight the characteristics and functionalities thereof when in use, and in which in particular:
- Figure 1 is a side view of the brace of the invention worn on a hand;
- Figure 2 is a view of the brace of Figure 1 seen from above of the back of the hand;

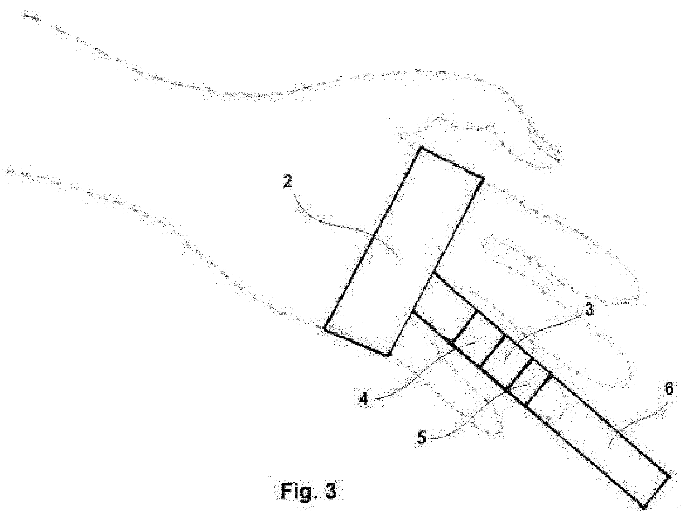

- Figure 3 is a view of the brace of Figure 1 seen from below of the palm of the hand.

With reference to the aforementioned figures, a brace according to the invention is generically indicated with 1 and consists of a semi-rigid support shaped substantially like a "T", which has a first horizontal branch 2 adapted for fixing around the hand in the metacarpal area and a second vertical branch 3, substantially perpendicular to the first, adapted for supporting any finger of the hand that has suffered an injury, except for the thumb, and that is in need of being immobilised for corrective or therapeutic purposes.

The brace can be made from various materials, provided that they are semi-rigid and shapeable, i.e. suitable for creating an adequate support for the injured finger without however compressing it or creating needless rigidity around the hand, and at the same time advantageously allowing the support to be shaped around the injured finger to adapt it to the size of each different finger of the hand and of each patient of different age and size. According to a particular embodiment of the present brace, the semi-rigid support is made with a material able to be manually cold-deformed, so that an operator can easily bend the support and shape it as desired, according to requirements. For example, such a semi-rigid support can be made with a material consisting of one or more sheets of aluminium, coated on one or both surfaces, inner and outer, by foamed material, preferably selected from polyethylene, elastic foamed polyurethane, polyurethane foam, and combinations thereof. For a greater comfort of the patient, all of the inner surfaces of the present brace, intended for making contact with the skin of the patient, can also be coated with a soft material, preferably a soft breathable fabric.

Thanks to its T-shape, the brace of the invention can advantageously be used to immobilise any finger of the hand, except the thumb, by simply sliding the horizontal branch 2 around the hand until the vertical branch 3 comes into contact with the lower surface of the finger to be immobilised; only at this moment the horizontal branch 2 is fixed around the hand at the metacarpal area and the same is done for the vertical branch 3, which is blocked around the injured finger to be immobilised. Both the horizontal branch 2 and the vertical branch 3 of the present brace are preferably shaped so as to follow the anatomy of the hand and thus have reduced impact on the patient often forced to wear this type of brace even for long time periods.

The application of the present brace foresees that, once the support is fixed under the finger, the part of the support in excess is folded over the finger to cover and give further protection to the area covered by the injury; any possible further remaining parts in excess can be easily removed by cutting them with scissors, with the end result that the finger to be immobilised is held effectively without being excessively compressed and without the hand being covered by the device for a surface that is too large and without it being needlessly impeded in movement.

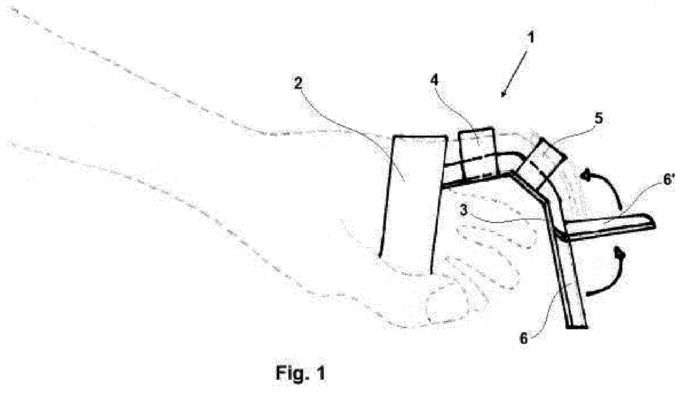

The fixing both of the horizontal branch 2 and of the vertical branch 3 of the brace is carried out through suitable anchoring means, which can for example be straps equipped with Velcro® portions for quick opening/closing and adjustment, intended to insert in suitable loops in the support. With reference to Figure 1, for example, the brace of the invention can comprise two straps 4 and 5 for fixing the vertical branch 3 to the finger to be immobilised; in general, the present brace comprises at least one anchoring means of the vertical branch of the brace to the finger to be immobilised, and an anchoring means of the horizontal branch 2 to the metacarpal area of the hand.

According to the specific needs of the patient and the size of the finger to be immobilised, the vertical branch 3 of the present standard sized brace can be more or less long with respect to the length of the finger to be immobilised; thus, the part of the branch 3 in excess of the length of the finger, at the time of application, is folded back to cover the upper part of the finger, thus creating a further useful further protection. An excess portion of the branch 3 can in any case be cut away with scissors. With reference to the attached Figure 1, the part of the branch 3 in excess of the length of the finger, that is thus folded back, is indicated with 6; such a part in excess at the time of folding back, is indicated with 6'. Such an excess part 6', once folded back over the finger to be immobilised, can be kept in position using at least one of the two straps 4 and 5 for fixing the vertical branch 3 to the finger.

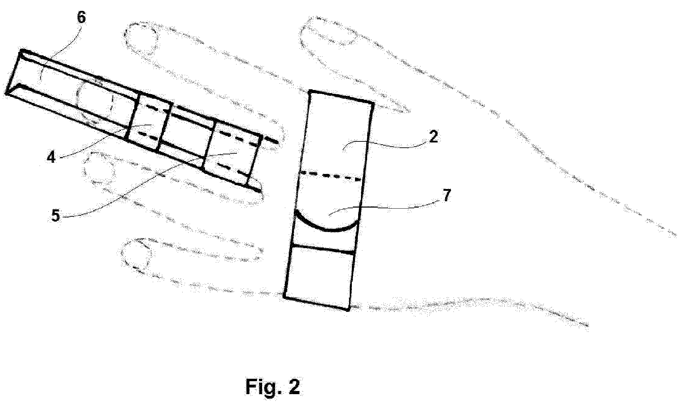

With particular reference to the attached Figure 2, an example of anchoring means of the horizontal branch 2 of the present brace is shown, made with a strap 7 that fixes such a branch to the metacarpal area of the hand. Such a strap 7 can for example be made so as to insert into a suitable loop on the branch 2 and close on the branch 2 itself through portions made from Velcro^{®}.

There are many advantages of the brace according to the invention. Its particular configuration firstly allows valid immobilisation of the fractures of the fingers - of the phalanges, either proximal or medial - of all of the fingers except the thumb, and also allows effective immobilisation even in cases of tendon injuries since an optimal hyper-extension and hyper-flexion is obtained. The present brace also constitutes a valid protection in the case of wounds or of post-surgery inflammation that involves the fingers, with particular ease in managing even the presence of nail wounds or injuries. The functional recovery of the mobility of the finger is also quick, since the brace according to the invention blocks only the joint segment suffering from the injury. As well as the surprising advantages in terms of clinical results and comfort of the patient, particularly important if the patient is of paediatric age, the brace of the invention can also be applied by a single health worker and extremely quickly, also being able to be removed and reapplied easily when needed, for example to medicate wounds or stitches, even at home, without the need for healthcare or medical interventions.

The brace according to the invention, therefore, offers clear advantages with respect to known orthotic devices, firstly due to the reduced impact, much better tolerated by the patient especially if they are of paediatric age, but also due to the positive impact on the time and method of application that, overall, also involve a certain economic saving. From this point of view, finally, it is worth noting the advantage of being able to decrease the stock of hand braces, since the present brace has great versatility and, unlike known devices, can be used for many types of injuries.

The invention is not limited to the embodiment described and illustrated above, but comprises any variant embodiment thereof.

## Claims

1. A brace (1) for immobilising any finger of a hand, other than the thumb, with corrective or therapeutic function of said finger following trauma, fractures, sprains, and similar, consisting of a semi-rigid support having substantially a T shape, comprising a first horizontal branch (2) adapted for fixing around the hand in the metacarpal area and a second elongated vertical branch (3), substantially perpendicular to said first horizontal branch (2), adapted for supporting said any finger, each of said first branch (2) and said second branch (3) being equipped with at least one fixing means to make it adhere, respectively, to said metacarpal area of the hand and to said finger.

2. The brace according to claim 1, wherein said fixing means consist of a strap equipped with Velcro and a loop in which said strap inserts.

3. The brace according to claim 1, wherein said first horizontal branch (2) is equipped with a strap (7) for fixing to the metacarpal area of the hand, and said second vertical branch (3) is equipped with two straps (4) and (5) for fixing to the finger to be immobilised.

4. The brace according to claim 1, wherein said support is made from a semi-rigid material internally coated, on the surface intended to make contact with the patient's skin, with a soft breathable fabric.

5. The brace according to claim 4, wherein said semi-rigid material is a material able to be cold-deformed manually.

6. The brace according to claim 5, wherein said semi-rigid material consists of one or more sheets of aluminium coated on one or on both surfaces, inner and outer, by a foamed material.

7. The brace according to claim 6, wherein said foamed material is selected from polyethylene, elastic foamed polyurethane, polyurethane foam, and combinations thereof.

8. The brace according to any one of the previous claims, wherein said horizontal branch (2) and/or said vertical branch (3) are shaped so as to follow the anatomy of the human hand for a reduced impact on the patient.

9. The brace according to any one of the previous claims, wherein a part (6) of said second vertical branch (3) exceeding the length of the finger to be immobilised is folded back over the finger itself to create a further protection.

10. The brace according to claim 9, wherein said exceeding part of the second vertical branch (3), once folded over the finger to be immobilised, is fixed to it by means of at least one fixing means of said vertical branch.
